(19) **Europäisches Patentamt**

**European Patent Office**

**Office européen des brevets**

(11) **EP 0 617 976 B1**

(12) **EUROPÄISCHE PATENTSCHRIFT**

(45) Veröffentlichungstag und Bekanntmachung des Hinweises auf die Patenterteilung:
**12.01.2000 Patentblatt 2000/02**

(51) Int. Cl.$^7$: **A61M 11/02**, A61M 15/00

(21) Anmeldenummer: **94102985.2**

(22) Anmeldetag: **28.02.1994**

(54) **Vorrichtung für die Erzeugung von Aerosolpulsen**

Device to produce aerosol jet

Dispositif pour la production de jets d'aérosol

(84) Benannte Vertragsstaaten:
**AT BE CH DE DK ES FR GB GR IT LI NL SE**

(30) Priorität: **31.03.1993 DE 4310575**

(43) Veröffentlichungstag der Anmeldung:
**05.10.1994 Patentblatt 1994/40**

(73) Patentinhaber:
**PARI GmbH Spezialisten für effektive Inhalation**
**82319 Starnberg (DE)**

(72) Erfinder:
• **Jaser, Stefan**
**D-86399 Bobingen (DE)**

• **Knoch, Martin, Dr.**
**D-82335 Berg (DE)**

(74) Vertreter:
**Zangs, Rainer E., Dipl.-Ing. et al**
**Hoffmann Eitle,**
**Patent- und Rechtsanwälte,**
**Arabellastrasse 4**
**81925 München (DE)**

(56) Entgegenhaltungen:
**EP-A- 0 178 925        CH-A- 625 718**
**GB-A- 2 164 569        US-A- 4 094 317**

• **PNEUMOLOGIE Bd. 44, 1990, Sonderheft Nr. 2, Seite 1026 BEINERT T. ET AL**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Xerox (UK) Business Services
2.16.7/3.6

**Beschreibung**

Die Erfindung betrifft eine Vorrichtung für die Erzeugung von Aerosolpulsen zur Aerosolapplikation bei der Lungenfunktionsdiagnostik und - therapie.

Bei der therapeutischen oder diagnostischen Anwendung von Aerosolpulsen, die während eines tiefen Einatmungsvorgangs des Patienten in die Atemluft eingeschossen werden, ist die Schärfe des Impulses von entscheidender Bedeutung. Denn bei der Therapie kann nur bei sehr scharfen Aerosolpulsen die im Aerosolpuls enthaltene Wirkstoffmenge gezielt in vorbestimmten Lungenbereichen deponiert werden. Bei der Diagnostik wird die Festlegung eines genauen Applikationszeitpunktes angestrebt, damit für eine Beurteilung des Exhalats eine verläßliche Ausgangs- und Bezugsgröße gegeben ist.

Zur Verdeutlichung der Einsatzmöglichkeiten von Aerosolpulsen in Therapie und Diagnostik wird im folgenden beispielhaft eine Diagnoseanwendung ausführlicher beschrieben, um daran die Anforderungen zu erläutern, die an die Größe und die zeitliche Verteilung des Aerosolpulses gestellt werden.

Zur Diagnose obstruktiver Ventilationsstörungen kann die Lungenfunktion im Wege der Analyse exhalierter Aerosolboli überprüft werden, wie von T.Beinert et al in Pneumologie 44 (1990), Sonderheft 2, 1026 beschrieben wird. Dabei wird ausgenutzt, daß sich monodisperse, inerte Aerosolteilchen mit einem vorgegebenen kleinen Durchmesser weitgehend wie ein nicht diffundierendes Gas verhalten und daher als Marker für den konvektiven Gastransport eingesetzt werden können. Wird ein solches Aerosol als kleinvolumiger Bolus, z.B. 20 $cm^3$ in die Atemluft eingeschossen und inhaliert, so kann man aus dem Volumen, über das die Aerosolteilchen im Exhalat verteilt sind, den konvektiven Austausch von Luftvolumina zwischen intrapulmonaler Reserveluft und Inspirat ablesen. Form und Lage des exhalierten Bolus sind gegenüber dem inhalierten verändert. Die Messung der Partikelkonzentration als Funktion des geatmeten Volumens erfolgt mittels eines kontinuierlich registrierenden Aerosolphotometers unter Verwendung eines monodispersen Aerosols aus zum Beispiel inerten Sebacinsäure-bis(2-äthylhexylester)-Tröpfchen (DEHS).

Die bekannte Messanordnung umfaßt einen Ventilblock, in dem ein Atemkanal, ein Luftkanal und ein Aerosolkanal vorgesehen sind. Durch den Atemkanal erfolgt die Ein- und Ausatmung, durch den Luftkanal wird Frischluft und durch den Aerosolkanal das Aerosol zugeführt. Im Ventilblock sind ferner Steuerventile untergebracht, die die Zuführung von Luft und Aerosol in der Atemluft steuern. Die Steuerventile für Luft und Aerosol sind mechanisch, elektromagnetisch oder pneumatisch betätigbar, damit während einer Einatmungsphase die Zuführung von Frischluft unterbrochen und kurzzeitig durch die Zuführung von Aerosol ersetzt werden kann. Durch diese kurze Umschaltung wird ein Aerosolpuls in die Atemluft eingeschossen.

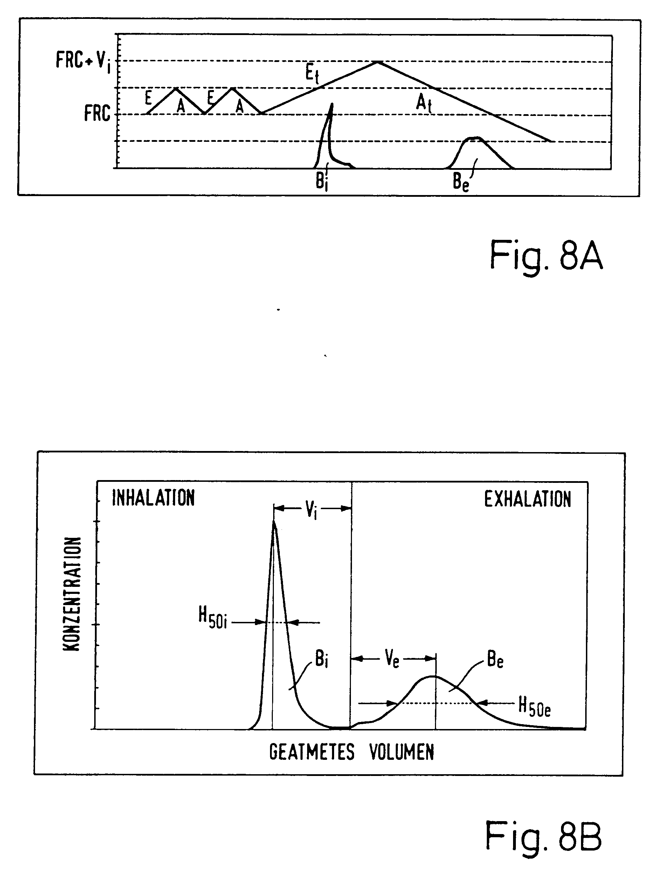

Ein typisches Atemmanöver ist in Fig. 8A dargestellt; ansteigende Kurvenabschnitte entsprechen den Einatmungsphasen E, absteigende Kurvenabschnitte den Ausatmungsphasen A. Während einer tiefen Einatmungsphase $E_t$ wird ein vorgegebenes Luftvolumen $V_i$ (z.B. 1 l) mit einem vorgegebenen Volumenfluß (z.B. 250 $cm^3$/s) von FRC aus inhaliert. In dieser Phase wird ein Aerosolbolus $B_i$ in die Atemluft eingebracht und das Aerosol vom Patienten inhaliert. In der tiefen Ausatmungsphase $A_t$, die sich an die tiefe Einatmungsphase anschließt, erscheint ein Aerosolbolus $B_e$ in der ausgeatmeten Luft.

Für die Analyse wird, wie in Fig. 8B schematisch gezeigt ist, der exhalierte Bolus $B_e$ mit dem inhalierten Bolus $B_i$ verglichen und zur Charakterisierung die Dispersion, Lageverschiebung und Form des exhalierten Bolus bestimmt. Die Dispersion H wird über ein Verhältnis der Halbwertsbreiten $H_{50}$ des inhalierten und des exhalierten Bolus entsprechend

$$H = ( H_{50e}{}^2 - H_{50i}{}^2 )^{1/2}$$

ermittelt. Die Dispersion ist ein Maß für die Vergrößerung des Volumens, über das die Aerosolteilchen im Exhalat verteilt sind, und beschreibt den konvektiven Austausch zwischen intrapulmonaler Reserveluft und Inspirat. Die Lageverschiebung M des exhalierten Bolus wird ebenfalls in Bezug auf den in das Inspirat eingeschossenen Aerosolbolus über

$$M = V_e - V_i$$

bestimmt. Während für den inhalierten Bolus von einem symmetrischen Konzentrationsverlauf ausgegangen wird, werden Asymmetrien des exhalierten Bolus über Formänderungsfaktoren ausgewertet.

Dieses Beispiel macht deutlich, in welchem Maße Therapie und Diagnostik von der Qualität des Aerosolpulses abhängen, der in die Atemluft eingeschossen wird. Insbesondere die Schärfe, d.h. die Flankensteilheit und maximal erreichte Konzentration des Aerosolpulses spielen dabei eine herausragende Rolle.

Das Dokument US-A-4 094 317 offenbart eine Vorrichtung für die Erzeugung von Aeropulsen, die die Merkmale des Oberbegriffs des Anspruchs 1 beinhaltet.

Die Aufgabe der Erfindung besteht darin, eine Vorrichtung für die Erzeugung von Aerosolpulsen zu schaffen, die sich aufgrund der erreichten Schärfe des Aerosolimpulses hervorragend für die Aerosolapplikation bei der Lungenfunktionsdiagnostik und - therapie eignet.

Diese Aufgabe wird gelöst durch eine Vorrichtung für die Erzeugung von Aerosolpulsen mit einem Ventilblock, in dem ein Atemkanal, ein Luftkanal, der quer in den Atemkanal mündet, und ein Aerosolkanal, der quer in den Atemkanal mündet, ausgebildet sind und Steuerventile, die zur Steuerung der Zuführung der Luft und des Aerosols durch die Kanäle in dem Ventilblock angeordnet und mechanisch, elektromagnetisch oder pneumatisch betätigbar sind, wobei erfindungsgemäß die Vorrichtung sich dadurch auszeichnet, daß im Atemkanal eine Strömungsfigur im Bereich der Einmündungen des Luftkanals und des Aerosolkanals vorgesehen ist, daß die Längsachse des Luftkanals zur Erzeugung einer tangentialen Anströmung der Strömungsfigur seitlich zur Längsachse des Atemkanals versetzt liegt, und daß die Längsachse des Aerosolkanals zur Erzeugung einer tangentialen Anströmung der Strömungsfigur seitlich zur Längsachse des Atemkanals versetzt liegt.

Die Strömungsfigur weist einen sich verjüngenden Abschnitt auf, dessen Spitze in Richtung der Strömung im Atemkanal während eines Einatmungsvorgangs orientiert ist.

In einer vorteilhaften Ausgestaltung weist die Strömungsfigur einen erweiterten Abschnitt an der Spitze des sich verjüngenden Abschnitts auf.

In einer weiteren vorteilhaften Ausgestaltung weist der erweiterte Abschnitt einen Durchmesser auf, der kleiner als der maximale Durchmesser und größer als der minimale Durchmesser des sich verjüngenden Abschnitts ist.

In einer weiteren vorteilhaften Ausgestaltung ist der erweiterte Abschnitt kugelförmig oder elliptisch.

In einer weiteren vorteilhaften Ausgestaltung geht der sich verjüngende Abschnitt stetig in den erweiterten Abschnitt über.

In einer weiteren vorteilhaften Ausgestaltung weist die Strömungsfigur einen zylindrischen Abschnitt am der Spitze gegenüber liegenden Ende des sich verjüngenden Abschnitts auf, dessen Durchmesser gleich dem maximalen Durchmesser des sich verjüngenden Abschnitts ist.

In einer besonders vorteilhaften Ausgestaltung ist der maximale Durchmesser des sich verjüngenden Abschnitts kleiner als der Durchmesser des Atemkanals, daß ein flacher Träger in dem Atemkanal zur Halterung der Strömungsfigur vorgesehen ist, dessen Durchmesser im wesentlichen gleich dem Durchmesser des Atemkanals ist und der bogenförmige Schlitze in dem über die Strömungsfigur hinausgehenden Bereich aufweist, und daß eine dünne elastische Scheibe auf der der Strömungsfigur abgewandten Seite des Trägers vorgesehen ist, die zumindest aufgrund der elastischen Kräfte in einer Ruhestellung und während eines Einatmungsvorgangs die Schlitze verschließt.

In einer besonders vorteilhaften Ausgestaltung liegen die Längsachsen des Luftkanals bzw. des Aerosolkanals in einer Ebene und die Längsachse des Atemkanals verläuft senkrecht zu dieser Ebene.

In einer besonders vorteilhaften Ausgestaltung ist die Anordnung des Luftkanals und des Aerosolkanals in Bezug auf die Längsachse des Atemkanals symmetrisch.

In einer besonders vorteilhaften Ausgestaltung ist die Strömungsfigur in einer Hülse angeordnet, die in den Ventilblock einsteckbar ist und den Atemkanal bildet. Dabei weist der Ventilblock erste und zweite Bohrungen zur Aufnahme von Sicherungsbolzen für die Arretierung der Hülse auf.

In einer weiteren vorteilhaften Ausgestaltung weist der Ventilblock dritte und vierte Bohrungen für die Aufnahme von PTC-Heizelementen auf.

Im folgenden wird die Erfindung anhand von Ausführungsbeispielen unter Bezugnahme auf die Zeichnungen genauer beschrieben. In den Zeichnungen zeigt:

Fig. 1 eine perspektivische Ansicht des Grundaufbaus eines Ventilblocks eines ersten Ausführungsbeispiels der erfindungsgemäßen Vorrichtung in einer vereinfachten Darstellung;

Fig. 2 eine weitere perspektivische, aber teilweise geschnittene Ansicht des Ventilblocks aus Fig. 1 mit zusätzlichen Einzelheiten;

Fig. 3 eine Querschnittsansicht des Ventilblocks aus Fig. 1 und 2 mit zusätzlichen Einzelheiten in Verbindung mit einem Ventil-Schaltschema;

Fig. 4 ein Diagramm des zeitlichen Verlaufs eines typischen Atemmanövers und korresponierende Schaltzustände der Ventile aus Fig. 3;

Fig. 5 eine Messkurve des Konzentrationsverlaufs eines mit herkömmlichen Mitteln und eines mit der erfindungsgemäßen Vorrichtung erzeugten Aerosolpulses;

Fig. 6 eine Querschnittansicht eines zweiten Ausführungsbeispiels der erfindungsgemäßen Vorrichtung;

Fig. 7 eine Querschnittansicht der Strömungsfigur des ersten und zweiten Ausführungsbeispiels; und

Fig. 8A und 8B Diagramme zur Erläuterung eines bekannten Anwendungsbeispiels aus dem Bereich der Lungenfunktionsdiagnostik.

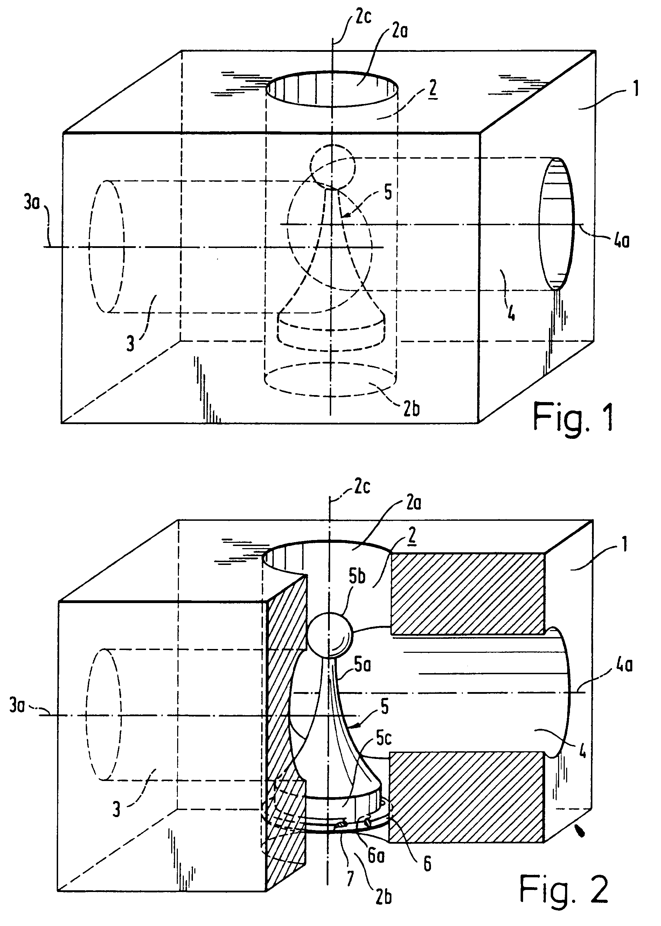

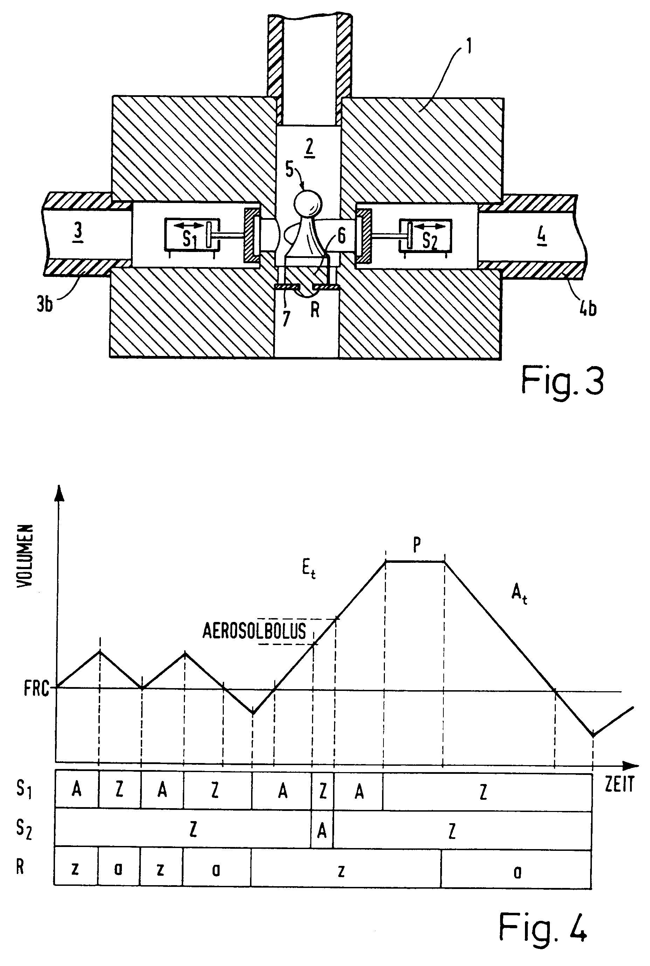

Anhand der Fig. 1 bis 3 wird ein erstes Ausführungsbeispiel der erfindungsgemäßen Vorrichtung beschrieben. In einer sehr vereinfachten Darstellung zeigt Fig. 1 einen Ventilblock der erfindungsgemäßen Vorrichtung, der allen Ausführungsbeispielen gemeinsam ist und als Hauptbestandteil der erfindungsgemäßen Vorrichtung angesehen werden kann. Fig. 2 zeigt den Ventilblock aus Fig. 1 in einer teilweise geschnittenen Darstellung. In Fig. 3 schließlich ist ein Schaltschema gezeigt, das den Ventilblock in der pneumatischen Verschaltung mit Zuleitungen und Ventilen wiedergibt. Auf Einzelheiten wurde in den Fig. 1 bis 3 so weit wie möglich verzichtet, um die Darstellung nicht zu überlasten und um den grundsätzlichen Aufbau zu verdeutlichen.

In Fig. 1 bezeichnet das Bezugszeichen 1 einen Ventilblock, an dem die Zuleitungen (nicht dargestellt) für die Zuführung und die Ventile (ebenfalls nicht dargestellt) für die Steuerung der zugeführten Luft und des zugeführten Aerosols angebracht werden können. Vertikal verläuft ein Atemkanal 2, dessen obere Öffnung 2a als Anschlußpunkt für die Zuleitung dient, durch die der Patient ein- bzw. ausatmet. Während beim Ausatmen die Atemluft aus der unteren Öffnung 2b des Atemkanals 2 ausströmt, ist beim Einatmen die Öffnung 2b verschlossen. Zur Realisierung dieser Funktion eignet sich beispielsweise ein einfaches Rückschlagventil; es kann aber auch ein gesteuertes elektromagnetisches oder pneumatisches Ventil an der Öffnung 2b des Atemkanals vorgesehen werden.

Quer zum Atemkanal 2 und vorzugsweise senkrecht dazu verläuft ein Luftkanal 3, der im Atemkanal 2 mündet und durch den die für den Atmungsvorgang benötigte Luft zum Atemkanal 2 zugeführt wird. Die Längsachse 3a des Luftkanals liegt seitlich neben der Längsachse 2c des Atemkanals. Ebenfalls quer zum Atemkanal 2 und ebenfalls vorzugsweise senkrecht dazu verläuft ein Aerosolkanal 4, der im Atemkanal 2 mündet und durch den das Aerosol zugeführt wird. Wie beim Luftkanal 3 liegt die Längsachse 4a des Aerosolkanals seitlich neben der Längsachse 2c des Atemkanals, jedoch auf der dem Luftkanal gegenüber liegenden Seite des Atemkanals, wie in Fig.1 gezeigt ist. Der Luftkanal 3 und der Aerosolkanal 4 sind zumindest so angeordnet, daß sie im wesentlichen auf gleicher Höhe in den Atemkanal 2 einmünden. Bei auf den Atemkanal bezogener senkrechter Anordnung sowohl des Luftkanals 3 als auch des Aerosolkanals 4 liegen die Längsachsen 3a und 4a des Luft- bzw. Aerosolkanals in einer Ebene. Vorzugsweise ist die Anordnung der drei Kanäle sysmmetrisch zur Längsachse 2c des Atemkanals, wie in Fig.1 dargestellt. Das bedeutet, es existieren drei zueinander parallele Ebenen, wobei in der ersten Ebene die Längsachse 3a des Luftkanals, in der zweiten die Längsachse 2c des Atemkanals und in der dritten die Längsachse 4a des Aerosolkanals liegt.

Bezugszeichen 5 bezeichnet eine rotationssymmetrische Strömungsfigur, die die im Atemkanal 2 entstehenden Luft- und Aerosolströmungen beeinflußt. Die Strömungsfigur 5 besitzt einen sich verjüngenden Abschnitt 5a und einen erweiterten Abschnitt 5b am spitzen Ende des sich verjüngenden Abschnitts. Der sich verjüngende Abschnitt 5a besitzt einen maximalen Durchmesser $d_{5amax}$ und einen minimalen Durchmesser $d_{5amin}$, die kontinuierlich in einander übergeführt werden. Der maximale Durchmesser $d_{5amax}$ ist kleiner als der Durchmesser des Atemkanals 2. Der maximale Durchmesser $d_{5bmax}$ des erweiterten Abschnitts 5b ist größer als der minimale Durchmesser $d_{5amin}$, aber kleiner als der maximale Durchmesser $d_{5amax}$ des sich verjüngenden Abschnitts 5a der Strömungsfigur. Der erweiterte Abschnitt 5b ist vorzugsweise eine Kugel, wie in Fig.1 gezeigt ist, oder ein Ellypsoid. Der Übergang zwischen dem sich verjüngenden Abschnitt 5a und dem erweiterten Abschnitt 5b kann unstetig sein, wie in Fig. 1 gezeigt ist. Der sich verjüngende Abschnitt 5a kann aber auch stetig in den erweiterten Abschnitt 5b übergeführt werden. Am breiten Ende des sich verjüngenden Abschnitts 5a kann ein zylindrischer Abschnitt 5c mit einem Durchmesser $d_{5c}$ vorgesehen sein, der gleich dem maximalen Durchmesser $d_{5amax}$ des sich verjüngenden Abschnitt 5a ist.

Die Strömungsfigur 5 ist im Atemkanal 2 an einer Stelle angeordnet, die in der Höhe der Einmündungen des Luft- und Aerosolkanals liegt. Die Spitze des sich verjüngenden Abschnitts 5a weist auf die Öffnung 2a des Atemkanals und somit in Richtung der während des Einatmungsvorgangs bestehenden Strömung. Wegen der versetzten Lage der Zuführkanäle 3 und 4 wird die Strömungsfigur 5 von der zugeführten Luft bzw. dem zugeführten Aerosol tangential im Bereich des sich verjüngenden Abschnitt 5a angeströmt.

In Fig. 2, die den Ventilblock 1 in einer teilweise geschnittenen Darstellung zeigt, ist die seitliche Versetzung der Längsachsen 3a und 4a der Zuführkanäle 3 und 4 gegenüber der Längsachse 2a des Atemkanals 2, ebenso wie die Lage der Strömungsfigur 5 und des sich verjüngenden Abschnitts 5a in Bezug auf die Einmündungen der Zuführkanäle 3 und 4 deutlich erkennbar.

Aufgrund der Versetzung der Längsachsen strömt die durch den Luftkanal 3 herangeführte Luft bzw. das durch den Aerosolkanal 4 herangeführte Aerosol die Strömungsfigur 5 seitlich an, wodurch ein um die Strömungsfigur herum verlaufender Wirbel mit einer Rotationsgeschwindigkeit $V_{rot}$ entsteht. Gleichzeitig wird die Luft bzw. das Aerosol in axialer Richtung der Strömungsfigur 5 mit einer Axialgeschwindigkeit $V_{ax}$ bewegt, da der Patient durch den Atemkanal 2 einatmet.

Mit einer Strömungsfigur mit einem sich verjüngenden Abschnitt ist das Verhältnis von Axial- zu Rotationsgeschwindigkeit $V_{ax}/V_{rot}$ im Bereich der tangentialen Zuströmung annähernd konstant. Dadurch wird eine gleichmäßige Umlenkung der Strömung mit minimalen Verweilzeiten im Zuströmbereich erzielt.

Am Ende des sich verjüngenden Abschnitts 5a wird keine Energie mehr durch die tangentiale Zuströmung zugeführt. Dies führt an der Wand des Atemkanals 2 unmittelbar stromab der Zuführöffnung des Luftkanals 3 und des Aerosolkanals 4 zu einer Strömungsablösung mit Totwasserbildung. Die sich im Totwasser ringförmig ansammelnden Aerosolpartikel strömen nur langsam ab. Der erweiterte Abschnitt 5b bewirkt eine konvektive Beschleunigung der Strömung in diesem ablösegefährdeten Bereich, wodurch das Totwasser unterdrückt wird und ein schneller Abtransport der Aerosolpartikel gewährleistet ist.

Durch die zur Achse 2c des Atemkanals 2 symmetrische Anordnung der Zuströmkanäle 3 und 4 bleiben das Verhältnis von Axial- zu Rotationsgeschwindigkeit $V_{ax}/V_{rot}$ sowie der Drehsinn des Wirbels um die Strömungsfigur 5 unverändert, auch wenn die Zuströmung kurzzeitig vom Luftkanal 3 auf den Aerosolkanal 4 umgeschaltet wird. Übliche Einspeisetechniken würden eine Änderung des Strömungszustandes mit Ausbildung von veränderlichen Totwassergebieten zur Folge haben. Daraus würde ein verzögerter Transport des Aerosols in den Strömungsquerschnitten resultieren, der die Anstiegs- und Abstiegsflanken sowie den Maximalwert des Konzentrationssignals des Aerosolbolus abflacht.

Die ablösungsfreie Strömungsumlenkung sowie die konstanten Strömungsbedingungen bei der alternierenden Einspeisung über die Kanäle 3 und 4 bewirken eine steile Anstiegs- und Abstiegsflanke des Konzentrationssignals des Aerosolbolus. Die Strömungsfigur verringert zudem das Totraumvolumen im Atemkanal im Bereich der Einmündungen der Zuführkanäle, d.h. das Volumen, das beim Wechsel zwischen Luft- und Aerosolzuführung jeweils ausgeräumt werden muß.

Darüberhinaus ist in Fig. 2 dargestellt, daß die Strömungsfigur 5 im Atemkanal 2 an einem flachen Träger 6 befestigt ist, der am Fuß der Strömungsfigur 5 vorgesehen ist und der sich zur Innenwand des Atemkanals 2 erstreckt. Damit während des Ausatmens die Atemluft zur Öffnung 2b des Atemkanals gelangen kann, sind in dem Träger 6 kreisbogenförmige Schlitze 6a vorgesehen. Am zylindrischen Abschnitt 5c der Strömungsfigur vorbei strömt die Atemluft durch die Schlitze 6a hindurch zur Öffnung 2b des Atemkanals, wenn der Patient in dem Atemkanal 2 hinein ausatmet.

Auf der von der Strömungsfigur abgewandten Seite des Trägers 6 ist eine dünne elastische Scheibe 7 aus Kunststoff oder Gummi angeordnet, die einen Durchmesser besitzt, der im wesentlichen dem Durchmesser des Atemkanals 2 entspricht. Aufgrund der elastischen Kräfte liegt die Scheibe 7 in der Ruhestellung auf der Oberfläche des Trägers 6 auf und verschließt die Schlitze 6a; während des Einatmens werden die elastischen Kräfte der elastischen Scheibe 7 durch die sich einstellenden Druckverhältnisse unterstützt. Während des Ausatmens wird die elastische Scheibe 7 von der Oberfläche des Trägers 6 abgehoben und gibt die Schlitze 6a des Trägers frei, so daß die Atemluft aus der Öffnung 2b des Atemkanals ausströmen kann.

In Fig. 3 ist der Ventilblock 1 in einem Schaltschema integriert, das ein erstes und ein zweites pneumatisches Steuerventil S1 und S2 und ein Rückschlagventil R umfaßt. Das erste Steuerventil S1 ist im Luftkanal 3 angeordnet und verschließt bzw. öffnet die Einmündung des Luftkanals 3 in den Atemkanal 2. Gleichermaßen ist das zweite Steuerventil S2 im Aerosolkanal 4 angeordnet und verschließt bzw. öffnet die Einmündung des Aerosolkanals 4 in den Atemkanal 2. Nicht dargestellt sind die Leitungen, über die die beiden Steuerventile jeweils an eine pneumatische Steuerung zur zeitgesteuerten Betätigung angeschlossen sind. In der Mitte der Fig. 3 sind die Strömungsfigur 5, unterhalb davon der flache Träger 6, der hier einstückig mit dem Ventilblock 1 ausgebildet ist, und die dünne elastische Scheibe 7 gezeigt, die das Rückschlagventil R bildet. Über Zuleitungen 3b und 4b wird Luft dem Luftkanal 3 bzw. ein Aerosol dem Aerosolkanal 4 zugeführt.

Im folgenden wird vorausgesetzt, daß mit Hilfe eines Verneblers ein geeignetes Aerosol in ausreichender Menge bereitgestellt wird. Unter einem geeigneten Aerosol wird beispielsweise Tröpfchen- oder Partikelnebel von 10000 runden Tröpfchen bzw. Partikeln von 1 μm Durchmesser in 1 $cm^3$ Luft angesehen.

Anhand von Fig. 4 wird nun der Betrieb des ersten Ausführungsbeispiels erläutert.

Im oberen Teil der Fig. 4 ist das Atemmanöver dargestellt, das der Patient durchführt. Ansteigende Kurventeile der Atemkurve entsprechen Einatmungsphasen, abfallende Kurven hingegen Ausatmungsphasen. Während einer tiefen Einatmungsphase $E_t$ wird ein vorgegebenes Luftvolumen mit einem vorgegebenen Volumenfluß von FRC aus inhaliert. In dieser Phase wird ein Aerosolbolus in die Atemluft eingebracht und das Aerosol vom Patienten inhaliert. Nach einer Pause P folgt eine tiefe Ausatmungsphase $A_t$.

Im unteren Teil von Fig. 4 sind die Schaltzustände der beiden Steuerventile S1 und S2 sowie des Rückschlagventils R aufgeführt. Große Buchstaben kennzeichnen dabei die von der pneumatischen Steuerung erzwungenen Schaltzustände der Steuerventile, kleine Buchstaben die sich aufgrund der Druckverhältnisse ergebenden Schaltzustände des Rückschlagventils. Aus der Darstellung ergibt sich, daß das erste Steuerventil S1 so angesteuert wird, daß der Luftkanal entsprechend der Abfolge von Einatmungs- und Ausatmungsphasen offen (A) bzw. verschlossen (Z) ist. Das zweite Steuerventil S2 wird so angesteuert, daß der Aerosolkanal in der Regel verschlossen (Z) ist. Nur für die kurze Zeitspanne des Aerosolpulses werden während der tiefen Einatmungsphase die beiden Steuerventile S1 und S2 derart angesteuert, daß der Luftkanal verschlossen (Z) und der Aerosolkanal offen (A) ist.

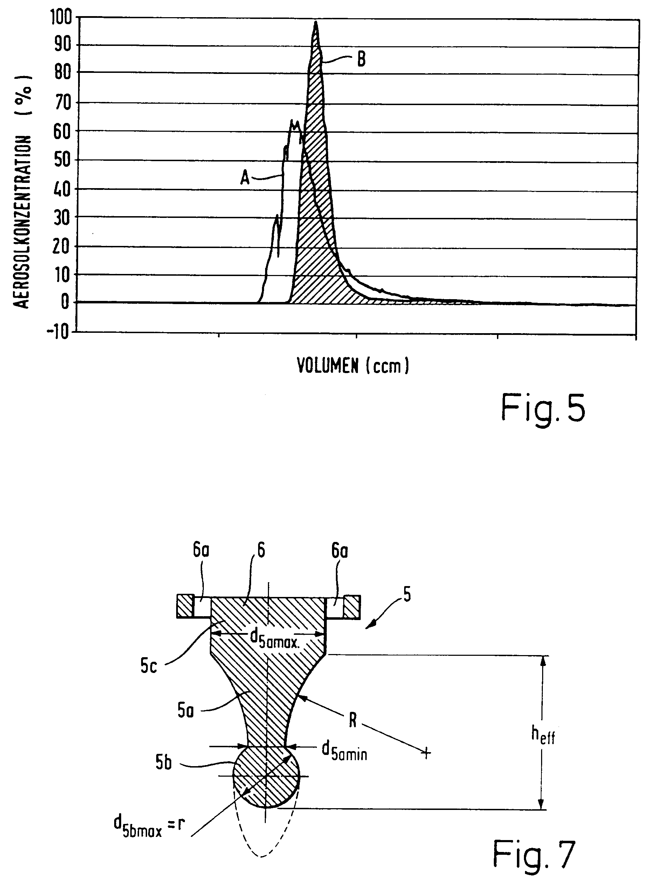

Da der Aerosolpuls, der mit Hilfe der erfindungsgemäßen Vorrichtung erzeugt werden kann, sehr steile Anstiegs- und Abstiegsflanken aufweist, ist die Zeitspanne der Bolus-Ansteuerung der beiden Steuerventile S1 und S2 vergleichsweise kurz, um 100% der Konzentration des bereitgestellten Aerosols zu erreichen. Bei der erfindungsgemäßen Vorrichtung sind etwa 0,08 s entsprechend einem Aerosolvolumen von ca. 20 cm$^3$ ausreichend, während bisher etwa 0,28 s (das 3,5-fache) erforderlich waren, um 100% der Ausgangskonzentration im Aerosolpuls zu erzielen. Diese Zusammenhänge sind in Fig. 5 dargestellt, die den Konzentrationsverlauf eines Aerosolpulses für eine herkömmliche Vorrichtung (A) und die erfindungsgemäße Vorrichtung (B) zeigt. Die Absizze des Diagramms zeigt das eingeatmete Volumen, die Ordinate die relative Aerosolkonzentration. Dabei ist zu beachten, daß die Flächen unter den beiden Kurven gleich sind, d.h. gleichen Volumina der Aerosolboli entsprechen. Aufgrund der hohen Flankensteilheit beträgt der Spitzenwert des Verlaufs B 100%, der Verteilung A jedoch nur 60% des Ausgangswerts.

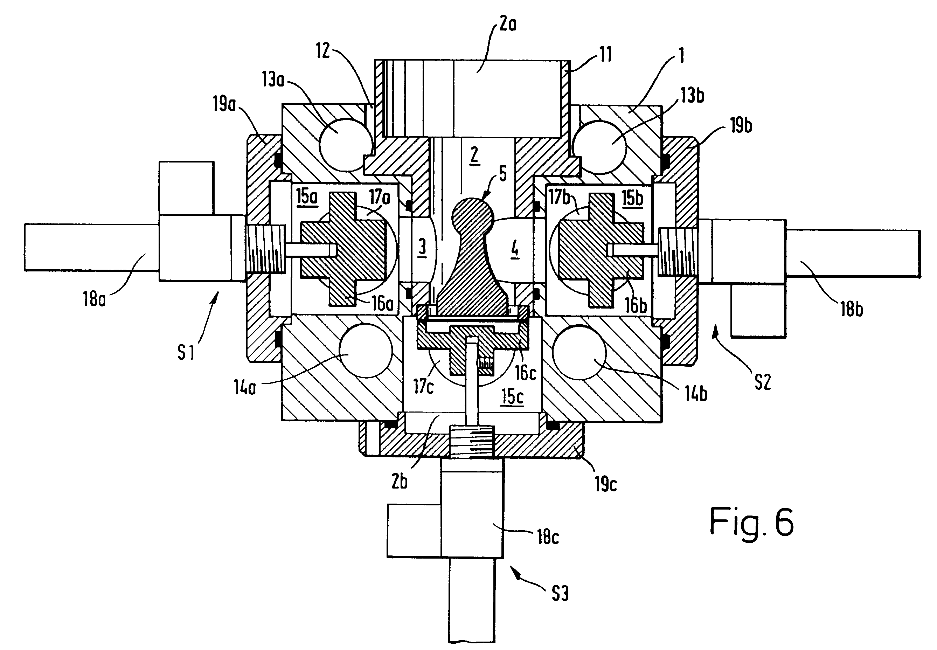

In Fig.6 ist ein zweites Ausführungsbeispiel der erfindungsgemäßen Vorrichtung in einer Querschnittsansicht gezeigt. Bei diesem Ausführungsbeispiel werden zahlreiche Einzelheiten der Anordnung und Ausgestaltung der Steuerventile, der Zuleitungen und eine besondere Ausbildung des Atemkanals erläutert.

In der Mitte der Fig.6 liegt die Strömungsfigur 5 im vertikal verlaufenden Atemkanal 2, der in einer einsteckbaren Hülse 11 ausgebildet ist. Im Ventilblock 1 ist eine Aufnahmebohrung 12 ausgebildet, in die die Hülse 11 eingesteckt ist. Für den Luftkanal 3 und den Aerosolkanal 4 weist die Hülse 11 an geeigneter Stelle Öffnungen auf. Da die Strömungsfigur 5 und auch das unterhalb davon vorgesehene Rückschlagventil fest mit der Hülse 11 verbunden sind, können die wesentlichen zu desinfizierenden Teile auf diese Weise einfach aus der Vorrichtung entnommen werden. Senkrecht zur Zeichnungsebene verlaufende, erste Bohrungen 13a und 13b im Ventilblock 1 sind zur Aufnahme von Sicherungsbolzen vorgesehen, die die Hülse 11 an den in die Bohrungen ragenden Kanten arretieren.

Senkrecht zur Zeichnungsebene verlaufende, zweite Bohrungen 14a und 14b im Ventilblock 1 sind zur Aufnahme von PTC-Heizelementen vorgesehen, mit denen der Ventilblock 1 zur Vermeidung von Kondensationseffekten beheizt wird.

Die Längsachse des Luftkanals 3 liegt vor der Zeichnungsebene der Fig.6, wohingegen die Längsachse des Aerosolkanals 4 hinter der Zeichnungsebene liegt, so daß beide Längsachsen nicht dargestellt sind. Beide Längsachsen liegen aber, wie beim ersten Ausführungsbeispiel, versetzt auf beiden Seiten des Atemkanals 2, so daß die Strömungsfigur 5 tangential im Bereich des sich verjüngenden Abschnitts angeströmt wird. Auf der vom Atemkanal abgewandten Seite münden die beiden Zuführkanäle 3 und 4 in einen ersten bzw. zweiten Raum 15a und 15b, in denen jeweils ein Ventilelement 16a und 16b eines ersten bzw. eines zweiten Steuerventils S1 und S2 angeordnet und bewegbar ist und in die senkrecht zur Zeichnungsebene verlaufende, dritte und vierte Bohrungen 17a und 17b im Ventilblock 1 münden, die als Zuleitungen für die Luft bzw. für das Aerosol dienen. Bewegt werden die beiden Ventilelemente 16a und 16b durch pneumatische Antriebseinheiten 18a und 18b, die außen am Ventilblock 1 angebracht sind und jeweils durch eine Abdeckung 19a und 19b für den ersten bzw. zweiten Raum 15a und 15b hindurch mit dem entsprechenden Ventilelement 16a und 16b verbunden sind.

Auch die Öffnung 2b des Atemkanals 2 ist beim zweiten Ausführungsbeispiel durch eine Abdeckung 19c verschlossen, durch die hindurch eine Antriebseinheit 18c eines dritten Steuerventils S3 mit einem zugehörigen Ventilelement 16c verbunden ist. Für die Atemluft während des Ausatmens mündet in einen dritten Raum 15c, in dem das Ventilelement 16c des dritten Steuerventils S3 angeordnet und bewegbar ist, eine senkrecht zur Zeichnungsebene verlaufende, fünfte Bohrung 17c im Ventilblock 1.

Der Betrieb des zweiten Ausführungsbeispiels entspricht im wesentlichen dem des ersten, der im Zusammenhang mit Fig. 4 beschrieben wurde. Das dritte Steuerventil S3 ist in der Regel geöffnet und wird nur während des tiefen Einatmungsvorgangs $E_t$ und der sich anschließenden Atmungspause P geschlossen.

In Fig. 7 ist beispielhaft eine Strömungsfigur 5 gezeigt. Anhand dieses Beispiels soll eine Möglichkeit der Formgebung, insbesondere des sich verjüngenden Abschnitts 5a erläutert werden. Die folgenden Verhältnisangaben beruhen auf Messreihen, die bei der Entwicklung der erfindungsgemäßen Vorrichtung mit senkrecht zum Atemkanal verlaufenden Zuführkanälen durchgeführt wurden, und stellen bevorzugte Größenordnungen dar. Jedoch sind diese Angaben in keinem Fall als Beschränkung aufzufassen.

Der sich verjüngende Abschnitt 5a der Strömungsfigur wird gebildet durch einen Kreisbogen, der am zylindrischen Abschnitt 5c ansetzt und einen Radius R besitzt. Das Verhältnis $R / d_{5amax}$ liegt vorzugsweise im Bereich zwischen 0,5 und 2,8. Der erweiterte Abschnitt 5b ist eine Kugel mit dem Radius r, der entsprechend einem Verhältnis r / R von 0,2 bis 0,7 gewählt wird. Die Lage des erweiterten Abschnitts 5b wird bestimmt durch die wirksame Höhe $h_{eff}$ der Strömungsfigur 5, die der Längserstreckung des sich verjüngenden Abschnitts 5a und des erweiterten Abschnitts 5b entspricht. Das Verhältnis $h_{eff}$ / R beträgt 0,85 bis 1,3.

Besonders vorteilhaft ist die Ausgestaltung des erweiterten Abschnitts 5b der Strömungsfigur 5 al rotationssymmetrischer Stromlinienkörper, der sich von seinem maximalen Durchmesser allmählich in Strömungsrichtung verjüngt (gestrichelte Kontur in Fig. 7).Diese Kontur kann auch durch einen Ellipsoid angenähert werden.

Die Strömungsfigur 5 gemäß Fig. 7 ist einstükkig mit dem flachen Träger 6 ausgebildet, über den die Strömungsfigur im Atemkanal befestigt ist. Gezeigt sind in Fig. 7 ferner die Schlitze 6a für die Atemluft während des Ausatmungsvorgangs.

**Patentansprüche**

1. Vorrichtung für die Erzeugung von Aerosolpulsen mit

   - einem Ventilblock (1), in dem

     -- ein Atemkanal (2),
     -- ein Luftkanal (3), der quer in den Atemkanal (2) mündet, und
     -- ein Aerosolkanal (4), der quer in den Atemkanal (2) mündet,

     ausgebildet sind, und

   - Steuerventile (S1, S2, S3, R), die zur Steuerung der Zuführung der Luft und des Aerosols durch die Kanäle (2, 3, 4) in dem Ventilblock (1) angeordnet und mechanisch, elektromagnetisch oder pneumatisch betätigbar sind, dadurch **gekennzeichnet**, daß

   - im Atemkanal (2) eine Strömungsfigur (5), die einen sich verjüngenden Abschnitt (5a) aufweist, dessen Spitze in Richtung der Strömung im Atemkanal (2) während eines Einatmungsvorgangs orientiert ist, im Bereich der Einmündungen des Luftkanals (3) und des Aerosolkanals (4) vorgesehen ist,

   - die Längsachse (3a) des Luftkanals (3) zur Erzeugung einer tangentialen Anströmung der Strömungsfigur (5) seitlich zur Längsachse (2c) des Atemkanals (2) versetzt liegt, und

   - die Längsachse (4a) des Aerosolkanals (4) zur Erzeugung einer tangentialen Anströmung der Strömungsfigur (5) seitlich zur Längsachse (2a) des Atemkanals (2) versetzt liegt.

2. Vorrichtung nach Anspruch 1, dadurch gekennzeichnet, daß die Strömungsfigur (5) einen erweiterten Abschnitt (5b) an der Spitze des sich verjüngenden Abschnitts (5a) aufweist.

3. Vorrichtung nach Anspruch 2, dadurch gekennzeichnet, daß der erweiterte Abschnitt (5b) einen Durchmesser aufweist, der kleiner als der maximale Durchmesser ($d_{5amax}$) und größer als der minimale Durchmesser ($d_{5amin}$) des sich verjüngenden Abschnitts (5a) ist.

4. Vorrichtung nach Anspruch 2 oder 3, dadurch gekennzeichnet, daß der erweiterte Abschnitt (5c) kugelförmig oder elliptisch ist.

5. Vorrichtung nach Anspruch 2, 3 oder 4, dadurch gekennzeichnet, daß der sich verjüngende Abschnitt (5a) stetig in den erweiterten Abschnitt (5c) übergeht.

6. Vorrichtung nach einem der Ansprüche 1 bis 5, dadurch gekennzeichnet, daß die Strömungsfigur (5) einen zylindrischen Abschnitt (5c) am der Spitze gegenüber liegenden Ende des sich verjüngenden Abschnitts (5a) aufweist, dessen Durchmesser gleich dem maximalen Durchmesser ($d_{5amax}$) des sich verjüngenden Abschnitts (5a) ist.

7. Vorrichtung nach einem der Ansprüche 1 bis 6, dadurch gekennzeichnet, daß der maximale Durchmesser des sich verjüngenden Abschnitts (5a) kleiner als der Durchmesser des Atemkanals (2) ist, daß ein flacher Träger (6) in dem Atemkanal (5) zur Halterung der Strömungsfigur (5) vorgesehen ist, dessen Durchmesser im wesentlichen gleich dem Durchmesser des Atemkanals (2) ist und der bogenförmige Schlitze (6c) in dem über die Strömungsfigur hinausgehenden Bereich aufweist, und daß eine dünne elastische Scheibe (7) auf der der Strömungsfigur abgewandten Seite des Trägers (6) vorgesehen ist, die zumindest aufgrund der elastischen Kräfte in einer Ruhestellung und während eines Einatmungsvorgangs die Schlitze (6a) verschließt.

8. Vorrichtung nach einem der Ansprüche 1 bis 7, dadurch gekennzeichnet, daß die Längsachsen (3a, 4a) des Luftkanals (3) bzw. des Aerosolkanals (4) in einer Ebene Liegen und die Längsachse (2c) des Atemkanals (2) senkrecht zu dieser Ebene verläuft.

9. Vorrichtung nach Anspruch 8, dadurch gekennzeichnet, daß die Anordnung des Luftkanals (3) und des Aerosolkanals (4) in Bezug auf die Längsaches (2c) des Atemkanals (2) symmetrisch ist.

10. Vorrichtung nach einem der Ansprüche 1 bis 9, dadurch gekennzeichnet, daß die Strömungsfigur (5) in einer Hülse (11) angeordnet ist, die in den Ventilblock (1) einsteckbar ist und den Atemkanals (2) umschließt, und der Ventilblock (1) erste und zweite Bohrungen (13a, 13b) zur Aufnahme von Sicherungsbolzen für die Arretierung der Hülse (11) aufweist.

11. Vorrichtung nach einem der Ansprüche 1 bis 10, dadurch gekennzeichnet, daß der Ventilblock (1) dritte und vierte Bohrungen (14a, 14b) für die Aufnahme von PTC-Heizelementen aufweist.

**Claims**

1. Appliance for generating aerosol pulses with

- one valve block (1) into which open

  -- one breathing channel (2),
  -- one air channel (3), opening transversely into the breathing channel (2), and
  -- one aerosol channel (4), opening transversely into the breathing channel (2)

  and

- control valves (S1, S2, S3, R) which are arranged in the valve block (1) to control the supply of air and of aerosol through channels (2, 3, 4) and can be actuated mechanically, electromechanically or pneumatically, **characterized** in that

- a flow body (5) having a tapering section (5a) whose tip points in the direction of the flow in the breathing channel (2) during an inhalation process is provided in the breathing channel (2) in the region into which the air channel (3) and the aerosol channel (4) open,

- the position of the longitudinal axis (3a) of the air channel (3) is displaced laterally relative to the longitudinal axis (2c) of the breathing channel (2) to generate a tangential flow against the flow body (5), and

- the position of the longitudinal axis (4a) of the aerosol channel (4) is displaced laterally relative to the longitudinal axis (2a) of the breathing channel (2) to generate a tangential flow against the flow body (5).

2. Appliance according to Claim 1, characterised in that the flow body (5) has an enlarged section (5b) at the tip of the tapering section (5a).

3. Appliance according to Claim 2, characterised in that the enlarged section (5b) has a diameter that is smaller than the maximum diameter ($d_{5amax}$) and larger than the minimum diameter ($d_{5amin}$) of the tapering section (5a).

4. Appliance according to Claim 2 or 3, characterised in that the enlarged section (5c) is spherical or elliptical.

5. Appliance according to Claim 2, 3 or 4, characterised in that the transition from the tapering section (5a) to the enlarged section (5c) is continuous.

6. Appliance according to one of the Claims 1 to 5, characterised in that the flow body (5) has, at the end of the tapering section (5a) opposite to the tip, a cylindrical section (5c) whose diameter is equal to the maximum diameter (d5amax) of the tapering section (5a).

7. Appliance according to one of the Claims 1 to 6, characterised in that the maximum diameter of the tapering section (5a) is smaller than the diameter of the breathing channel (2), in that there is provided a flat carrier (6) in the breathing channel (5) to support the flow body (5), whose diameter is essentially equal to the diameter of the breathing channel (2) and which has arch-shaped slots (6c) in the region extending beyond the flow body, and in that there is provided on the side of the support (6) facing away from the flow body a thin elastic disk (7) which, at least based on elastic forces, seals the slots (6a) in a resting position and during an inhalation process.

8. Appliance according to one of the Claims 1 to 7, characterised in that the longitudinal axes (3a, 4a) of the air channel (3) and of the aerosol channel (4) respectively lie in one plane and the longitudinal axis (2c) of the breathing channel (2) runs perpendicularly to this plane.

9. Appliance according to Claim 8, characterised in that the arrangement of the air channel (3) and of the aerosol channel (4) is symmetrical relative to the longitudinal axis (2c) of the breathing channel (2).

10. Appliance according to one of the Claims 1 to 9, characterised in that the flow body (5) is arranged in a sleeve (11) that can be plugged into the valve block (1) and surrounds the breathing channel (2), and the valve block (1) has first and second holes (13a, 13b) drilled in it to accommodate the securing bolts to lock the sleeve (11) in position.

11. Appliance according to one of the Claims 1 to 10, characterised in that the valve block (1) has third and fourth holes (14a, 14b) drilled in it to accommodate PTC (positive temperature coefficient) heating elements.

**Revendications**

1. Dispositif pour la production de jets d'aérosol, comportant

   - un bloc soupape (1), dans lequel sont réalisés

     -- un canal de respiration (2),
     -- un canal d'air (3), débouchant transversalement dans le canal de respiration (2), et
     -- un canal d'aérosol (4), débouchant transversalement dans le canal de respiration (2),

et

- des soupapes de commande (S1, S2, S3, R), qui sont disposées pour assurer la commande de l'alimentation en air et en aérosol par les canaux (2, 3, 4) dans le bloc de soupape (1) et sont actionnables, de façon mécanique électromagnétique ou pneumatique, caractérisé en ce que

- dans le canal de respiration (2) est prévue, dans la zone des embouchures du canal d'air (3) et du canal d'aérosol (4), une figure d'écoulement (5), qui présente un tronçon (5a) allant en s'effilant, dont la pointe est orientée dans la direction de l'écoulement dans le canal de respiration (2) pendant le processus d'inhalation,

- l'axe longitudinal (3a) du canal d'air (3) est disposé de façon décalée latéralement par rapport à l'axe longitudinal (2c) du canal de respiration (2), pour générer une arrivée d'écoulement tangentielle à la figure d'écoulement (5)

- l'axe longitudinal (4a) du canal d'aérosol (4) est disposé de façon décalée latéralement par rapport à l'axe longitudinal (2a) du canal de respiration (2), pour générer une arrivée d'écoulement tangentielle à la figure d'écoulement (5).

2. Dispositif selon la revendication 1, caractérisé en ce que la figure d'écoulement (5) présente un tronçon (5b) élargi, à la pointe du tronçon (5a) allant à s'effilant.

3. Dispositif selon la revendication 2, caractérisé en ce que le tronçon (5b) élargi présente un diamètre inférieur au diamètre maximal ($d_{5amax}$) et supérieur au diamètre minimal ($d_{5amin}$) du tronçon (5a) allant en s'effilant.

4. Dispositif selon la revendication 2 ou 3, caractérisé en ce que le tronçon (5c) élargi est en forme sphérique ou elliptique.

5. Dispositif selon la revendication 2, 3 ou 4, caractérisé en ce que le tronçon (5a) allant en s'effilant se transforme de façon continue en le tronçon (5c) élargi.

6. Dispositif selon l'une des revendications 1 à 5, caractérisé en ce que la figure d'écoulement (5) présente, à l'extrémité située à l'opposé de la pointe du tronçon (5a) allant en s'effilant, un tronçon (5c) cylindrique dont le diamètre est égal au diamètre maximal ($d_{5amax}$) du tronçon (5a) allant en s'effilant.

7. Dispositif selon l'une des revendications 1 à 6, caractérisé en ce que le diamètre maximal du tronçon (5a) allant en s'effilant est inférieur au diamètre du canal de respiration (2), en ce qu'est prévu dans le canal de respiration (5) un support (6) plat, pour fixer la figure d'écoulement (5), dont le diamètre est sensiblement égal au diamètre du canal de respiration (2) et qui présente des fentes (6c) en forme d'arcs dans la zone dépassant la figure d'écoulement, et en ce qu'est prévu sur la face, opposée à la figure d'écoulement, du support (6), un disque (7) élastique mince qui obture les fentes (6a), au moins du fait des efforts élastiques, en une position de repos et pendant un processus d'inhalation.

8. Dispositif selon l'une des revendications 1 à 7, caractérisé en ce que les axes longitudinaux (3a, 4a) du canal d'air (3) respectivement du canal d'aérosol (4) sont situés dans un plan et l'axe longitudinal (2c) du canal de respiration (2) s'étend perpendiculairement à ce plan.

9. Dispositif selon la revendication 8, caractérisé en ce que l'agencement du canal d'air (3) et du canal d'aérosol (4) est symétrique par rapport à l'axe longitudinal (2c) du canal de respiration (2).

10. Dispositif selon l'une des revendications 1 à 9, caractérisé en ce que la figure d'écoulement (5) est disposée dans une douille (11), qui est susceptible d'être enfichée dans le bloc de soupape (1) et qui entoure le canal de respiration (2), et le bloc de soupape (1) présente des premier et deuxième perçages (13a, 13b) destinés à recevoir des boulons de sécurité destinés à bloquer la douille (11).

11. Dispositif selon l'une des revendications 1 à 10, caractérisé en ce que le bloc de soupape (1) présente des troisième et quatrième perçages (14a, 14b), destinés à loger des éléments de chauffage à caractéristiques PTC.

Fig. 1

Fig. 2

Fig. 3

Fig. 4

Fig. 5

Fig. 7

Fig. 6

EP 0 617 976 B1

Fig. 8A

Fig. 8B